# EUROPEAN PATENT APPLICATION

(11) **EP 1 044 689 A1**
(43) Date of publication of application: **18.10.2000**
(21) Application number: 98961654.5
(22) Date of filing: 28.12.1998
(51) Int. Cl.: A61K 33/18, A61K 31/79, A61K 47/36

(54) **SOLID IODOPHOR PREPARATIONS AND PROCESS FOR PRODUCING THE SAME**

(30) Priority: 06.01.1998 JP 3190298; 16.06.1998 JP 16864798
(71) Applicant: FUJISAWA PHARMACEUTICAL CO., LTD., Osaka-shi, Osaka-fu 541-8514 (JP)
(72) Inventor: TAKAHASHI, Satoru, Nishinomiya-shi, Hyogo-ken/663-8203 (JP); SUGINO, Tatsuya, Ushiku-shi, Ibaraki-ken 300-1235 (JP); NAKAHARA, Tomoaki, Toshima-ku, Tokyo-to 170-0003 (JP); TAGUCHI, Yoshimi, Misuzu Shokai Ltd., Osaka-shi, Osaka-fu 541-0048 (JP); MURAKI, Nobuhiro, Riverson & Co., Ltd., Osaka-shi, Osaka-fu 541-0041 (JP); NAKAHARA, Tetsuya, Yamasan Company Ltd., Toshima-ku, Tokyo-to 170-0003 (JP); AGATA, Tomoyuki, Inasa-gun, Shizuoka-ken 431-1404 (JP); SASAKI, Yasoji, Yokohama-shi, Kanagawa-ken 233-0011 (JP)
(74) Representative: Gille Hrabal Struck Neidlein Prop Roos
(86) International application number: JP9806018
(87) International publication number: WO9934809

(57) **Abstract**

The present invention is a solid preparation of iodophor containing
(a) dextrin,
(b) iodides,
(c) oxidizing agents, and
(d) acids.

The above solid preparations of iodophor can efficiently provide a single-type solid preparation of iodophor which is stable, without releasing iodine during storage, and can rapidly be dissolved before use.

## Description

### Technical Field

This invention relates to a single-type solid preparation of iodophor to be dissolved before use. This solid preparation of iodophor is excellently stable without releasing iodine during storage, can rapidly be dissolved in water before use, and has various other advantages by using the solid preparation, not liquid preparation (easy to handle, not bulky, not in danger of liquid leakage during transportation, etc.). Thus, it is very useful as an iodine-type bactericide/disinfectant.

### Background Art

Iodine-type disinfectants exert their bactericidal effects under an acidic condition through iodine released. Iodine is only slightly soluble in water and has a sublimate property. Therefore, it is difficult to obtain a stable preparation of iodine by merely mixing solid iodine and solid surfactants. Then, it has been proposed that a mixture of iodides, oxidizing agents, and acids is dissolved in water to release iodine by chemical reaction in water.

Among the above components, a solid iodide and an oxidizing agent lie stably in the mixture of the two. However, once a powdered acid is added to the mixture under the ordinary handling condition, chemical reactions proceed gradually, and release sublimating iodine. Thus, the stability of the preparation is seriously deteriorated.

For this reason, a 'twin-type' of solid preparation of iodophor has been disclosed Japanese Unexamined Patent Publication No. 2-110. In this case, two separate preparations are prepared: one contains acids which interfere with the stability of the preparation and the other contains iodides and oxidizing agents, and these two preparations are mixed before use to be dissolved. However, it has some disadvantages of the 'twin-type' including inconvenience for use, poor solubility, etc.

Therefore, it has earnestly been desired that not a 'twin-type' but a 'single-type' of the solid preparation of iodophor which has excellent stability and increased solubility is provided.

### Disclosure of the Invention

The present invention was made in consideration of the above circumstances, and it is an object of the present invention to provide a 'single-type' solid preparation of iodophor which is stable under the ordinary storage conditions without releasing iodine and can easily be dissolved before use. It is a further object of the present invention to provide an efficient method for manufacturing the inventive preparation of iodophor.

The inventive solid preparation of iodophor that was able to achieve the above objects is characterized in containing:
(a) dextrin,
(b) iodides
(c) oxidizing agents, and
(d) acids.

In this preparation, the following is preferred modes of the present invention: the content of (a) dextrin, (b) iodides, (c) oxidizing agents, and (d) acids in the solid preparation of iodophor is 10 to 95%, 10 to 50%, 2 to 10%, and 5 to 50%, respectively; and a solid preparation of iodophor which contains potassium iodide as an iodide and potassium iodate as an oxidizing agent in a weight ratio of 4 or more : 1.

The methods for manufacturing the inventive solid preparation of iodophor that was able to achieve the above objects is the methods for manufacturing the solid preparation of iodophor containing (a) dextrin, (b) iodides, (c) oxidizing agents and (d) acids, which is characterized in
granulating the dextrin (a) and at least one component selected from the group consisting of iodides (b), oxidizing agents (c), and acids (d) under a wet condition produced by spraying water,
powdering the resultant mixed granulated product, and
mixing the powder product, if necessary, with at least one component selected from the group consisting of dextrin (a), iodides (b), oxidizing agents (c), and acids (d).

In the above method, the following are preferred modes of the present invention:
the method includes granulating the dextrin (a), together with iodides (b) and oxidizing agents (c) under a wet condition produced by spraying water,
powdering the resultant mixed granulated product, and
mixing the powder product with acids (d); and/or
the method includes granulating the dextrin (a), together with iodides (b) and/or oxidizing agents (c) under a wet condition produced by spraying water,
powdering the resultant mixed granulated product, and
mixing the powder product with acids (d) and, in addition, at least one component selected from the group consisting of dextrin (a), iodides (b), and oxidizing agents (c), if necessary.

In the above method, the following is also preferred of the present invention:
the content of (a) dextrin, (b) iodides, (c) oxidizing agents, and (d) acids in the solid preparations of iodophor is 10 to 95 %, 10 to 50 %, 2 to 10 %, and 5 to 50 %, respectively, by weight; and/or
the preparation contains potassium iodide as the (b) iodide and potassium iodate as the (c) oxidizing agent in a weight ratio of 4 or more:1.
In addition, it is preferred that the granulating step under a wet condition includes granulating at a temperature of intake air ranging from a room temperature to 120°C.

Furthermore, a solid preparations of iodophor that is a granulated powder containing povidone-iodide and dextrin, is included within the present invention.

In the above preparation,the following is preferred that:
the content of available iodine in the solid preparations of iodophor is 0.005 w/w% or more;and/or
the preparation contains the povidone-iodine and dextrin in a weight ratio of 90:10 to 5:95.

The above method for manufacturing solid preparations of iodophor is characterized in that the povidone-iodine and dextrin are granulated under a wet condition produced by spraying water. In the method, it is preferred that the granulated powder is obtained at a temperature of intake air ranging from a room temperature to 120°C.

### Brief Description of the Drawings

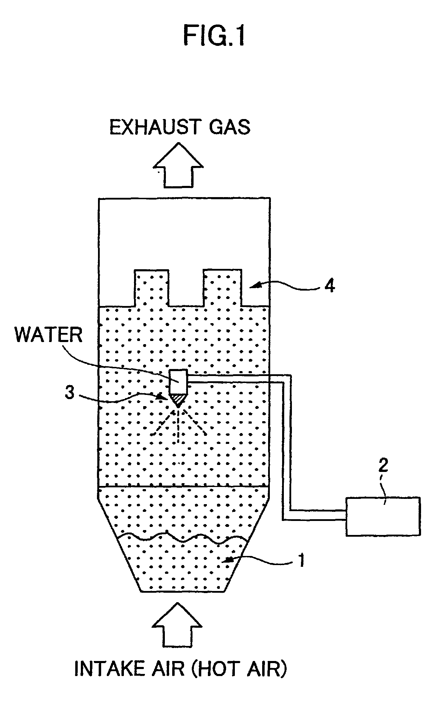

FIG. 1 shows a schematic diagram of the fluid bed granulation dryer used in the present invention.

### Description of the symbols

1. Powdered raw material
2. Liquid delivery pump
3. Spray nozzle
4. Bag filter

### Best Mode for Carrying Out The Invention

Considering the easiness of handling, etc., and paying attention to the 'single-type solid preparation of iodophor', the inventors have intensively investigated about increasing the stability and solubility of the solid preparation of iodophor. Therefore, they found that when dextrin is used as the iodine carrier, iodides and oxidizing agents can stably be retained in the dextrin without releasing iodine during storage, and that when water is sprayed on each component of the above preparation [the above (a) and at least one of (b), (c) and (d)] and when a liquid bed granulation dryer (for example, "Flow Coater Model FLO-5B" manufactured by Okawara Seisakusho, abbreviated as " "Flow Coater" hereinafter) is used for the preparation of the solid preparation, a desired solid preparation of iodophor with excellent stability and solubility can be provided, they accomplished the invention.

The most prominent features of the present invention are the use of dextrin as the iodine carrier as well as the water spraying on each component or their mixture during the granulatin and mixing process.

A 'single-type' of solid preparation of iodophor, which contains both polyvinylpyrrolidone (PVP) and dextrin as carriers as well as iodine and iodides, has also been disclosed heretofore Japanese Unexamined Patent Publication No. 9-100234. According to the above publication, however, there is a recognition that "dextrin, by itself alone, cannot lead to the formation of an excellently stable iodine complex". Accordingly, those inventors contemplated improvements of stability by mixing PVP or poly-N-vinylcaprolactam as an iodine carrier in addition with dextrin.

On the contrary, the present invention is clearly and constitutionally different from the invention disclosed in the above publication in that dextrin alone is used as the iodine carrier, and that PVP is not essentially contained. Although it has been believed, according to the above publication, that "when dextrin alone, without PVP, is used, an excellently stable iodine complex cannot be obtained", the manufacturing methods described in the present invention could unexpectedly provide an excellently stable iodophor preparation without releasing iodine during storage even when dextrin alone is used. This point carries the technical significance of the present invention.

The above methods of the present invention, that is, the method for preparation by spraying water on each component constituting the solid preparation of iodophor or their mixture and by using a liquid bed granulation dryer (Flow Coater), etc., has been found to be also extremely valid for manufacturing preparations comprising a povidone-iodine containing PVP and dextrin. Thus, it was shown that the granulated powder preparations containg povidone-iodine and dextrin prepared by the above manufacturing methods increase remarkably in water solubility and improve significantly in the bactericidal/disinfectant effect.

In the following, each component constituting the present invention is described.

First of all, solid preparations of iodophor which contain (a) dextrin, (b) iodides, (c) oxidizing agents, and (d) acids are described.

### (a) Dextrin

Dextrin is very important in the present invention as the component carrying iodine. Dextrin is obtained by hydrolyzing starch with an enzyme or acid, but commercially available products such as calcined dextrin and saccharified dextrin may be utilized.

For more effective exertion of the above carrier effect, the use of cyclodextrin or porous dextrin is preferred.

### (b) Iodides

There is no special restriction on the iodides to be used except that they must dissociate in the aqueous solution to release iodide ions. The examples of such iodides include alkaline metal iodides (sodium iodide, potassium iodide, etc.) and alkaline earth metal iodides (calcium iodide, magnesium iodide, etc.) as metallic iodides, and ammonium iodide, etc. as nonmetallic iodides. Among them, the use of alkaline metal iodides is recommended. Iodides obtained by reducing iodates (potassium iodate, sodium iodate, etc.) may be used.

### (c) Oxidizing agents

There is no special restriction on the oxidizing agents except that they must generate iodine molecules by reacting with the above iodides. The examples of such oxidizing agents include acids such as iodic acid, bromic acid, chromic acid, permanganic acid, peroxy acid (peroxytitanic acid, peroxynitric acid, peroxyphosphoric acid, etc.), iodine oxides (diiodine tetraoxide, diiodine pentaoxide, tetraiodine nonaoxide, etc.), halogenated isocyanuric acids (one to three hydrogens in isocyanuric acid are substituted by halogens including chlorine, iodine, and bromine, preferably by chlorine), etc. and their salts. The above salts include alkaline metal salts (sodium salts, potassium salts, etc.), alkaline earth metal salts (calcium salts, magnesium salts, etc.), non-metal salts (ammonium salts, amine salts, etc.), etc. Among them, alkaline metal salts are preferred, and sodium salts and potassium salts are more preferred.

In the concrete, the use of iodic acid, peroxy acid, halogenated isocyanuric acids, and their salts is recommended, and the use of iodates (sodium iodate, potassium iodate, etc.) as well as halogenated isocyanuric acids and their salts (sodium dichloroisocyanurate, trichloroisocyanuric acid, etc.) is especially preferred.

### (d) Acids

Acids to be used in this invention include ones, such as powdered acids; powdered acidic acid salts; lactones, carboxylic acid anhydrides, etc., that are soluble in water and hydrolyzed to acidic compounds. The examples of the powdered acids include saturated dicarboxylic acids (oxalic acid, malonic acid, succinic acid, etc.), unsaturated dicarboxylic acids (maleic acid, fumaric acid, etc.), oxy-acids (malic acid, tartaric acid, citric acid, etc.), inorganic acids (boric acid, pyrophosphoric acid, metaphosphoric acid, phosphorous acid, etc.), etc. The examples of the powdered acidic acid salts include acidic salts of inorganic acids such as hydrogen alkaline metal salts of inorganic acids (sodium dihydrogenphosphate, sodium hydrogensulfate, etc.) Among them, oxalic acid, citric acid, and alkaline metal hydrogensulfates (sodium salt, etc.) are preferred. The examples of lactones include glucono-delta-lactone, etc., and the examples of carboxylic acid anhydrides include succinic anhydride, maleic anhydride, etc.

The preferred content of these components in the solid preparation of iodophor is as follows: (a) dextrin: not less than 10% and not more than 95% (more preferably, not less than 20% and not more than 50%), (b) iodides: not less than 10% and not more than 50% (more preferably, not more than 20%), (c) oxidizing agents: not less than 2% and not more than 10% (more preferably, not more than 5%), and (d) acids: not less than 5% and not more than 50%. When a powdered base having an effervescent property is to be added to the preparation, it is preferred to appropriately adjust the amount of (d) in proportion to the amount of the base.

When potassium iodide is used as (b) the iodide and potassium iodate as (c) the oxidizing agent, it is recommended for the preparation to contain potassium iodide and potassium iodate in a weight ratio of 4 or more:1.

The above solid preparation of iodophor contains the (a) to (d) as essential components, and can also contain the following components unless they deteriorate the effect of the invention.

### Powdered bases having an effervescent property in reaction with acids

The powdered base means a powdered alkaline base (normal salts, acid salts, etc.). It is no special limitations to be used except that it has effervescence preperty in reaction with the (d) acids. In the concrete, they include carbonates (alkaline metal carbonates, etc. including sodium carbonate, potassium carbonate, ammonium carbonate, etc.), hydrogencarbonates (alkaline metal hydrogencarbonates, etc. including sodium hydrogencarbonate, potassium hydrogencarbonate, ammonium hydrogencarbonate, etc.), etc. In particular, the use of alkaline salts of hydrogencarbones (sodium salts, etc.) is recommended.

### Skin-protecting agents

The skin-protecting agents include ones such as hyaluronic acid, urea, chondroitin sulfate, chitin, chitosan, collagen, allantoin, and aloe gel as well as the following agents powered by using β-cyclodextrin: glycerin, sorbitol, poly(ethylene glycol), lanolin, squalene, etc.

Other components such as vapor absorbents (sodium sulfate, potassium sulfate, etc.), binders (mannitol, etc.), etc. may be added if necessary.

In the following, the methods of manufacturing the preparation are described.

For manufacturing the solid preparation of iodophor containing the (a) dextrin, (b) iodides, (c) oxidizing agents, and (d) acids, it is required that dextrin (a) and at least one component selected from the group consisting of iodides (b), oxidizing agents (c), and acids (d) are granulated under a wet condition produced by spraying water, and that the resultant mixed granulated product are powdered and mixed, if necessary, with at least one component selected from the group consisting of dextrin (a), iodides (b), oxidizing agents (c), and acids (d). In its essence, the most important point of this manufacturing methods lies in that the (a) and at least one component selected from (b), (c), and (d) are sprayed with water, followed by granulation and drying with the liquid bed granulation dryer (Flow Coater), etc. The methods make it possible to produce a single-type of a solid preparation of iodophor which is very excellent in the stability without releasing iodine during its storage and also excellent in the solubility even when dextrin alone is used as the carrier.

In the concrete, at first, dextrin (a) and at least one component selected from the group consisting of iodides (b), oxidizing agents (c), and acids (d) are granulated under a wet condition produced by spraying water to obtain a mixed granulated product. The resultant mixed granulated product may occur in the following three modes (① to ③).
① Mixed granulated products containing two components, that is, the (a) and another component selected from the (b), (c), and (d) [namely, mixed granulated products containing dextrin (a) and iodides (b); mixed granulated products containing dextrin (a) and oxidizing agents (c); and mixed granulated products containing dextrin (a) and acids (d)];
② Mixed granulated products containing three components, that is, the (a) and two other components selected from (b), (c), and (d) [namely, mixed granulated products containing dextrin (a), iodides (b), and oxidizing agents (c); mixed granulated products containing dextrin (a), iodides (b), and acids (d); and mixed granulated products containing dextrin (a), oxidizing agents (c), and acids (d)]
③ Mixed granulated products containing the four components (a), (b), (c), and (d) [namely, mixed granulated products containing dextrin (a), iodides (b), oxidizing agents (c), and acids (d)]

For the purpose of obtaining more excellent stability and solubility, it is most highly recommended to granulate iodides (b), oxidizing agents (c), and acids (d) separately, and then mix them. When the manufacturing efficiency, etc. are taken into consideration, however, a preferred mode of the mixture contains dextrin (a), and iodides (b) and/or oxidizing agents (c), and it is recommended to mix this mixture with acids (d) later.

As for the mixing step, either is acceptable whether to simply mix the appropriate components or to use an appropriate reaction mixture such as the oxidation-reduction reaction product of an iodide and an oxidizing agent (for example, potassium iodide and potassium iodate) as described in the below examples.

As the next step, the mixed granulated product is powdered, and if necessary, mixed with at least one component selected from the group consisting of dextrin (a), iodides (b), oxidizing agents (c), and acids (d). Thus, the desired solid preparation of iodophor is obtained. As noted above, the inventive solid preparation contains dextrin (a), iodides (b), oxidizing agents (c), and acids (d). Therefore, when mode ③ [a mixed granulated product containing all four components of (a), (b), (c), and (d)] is adopted as an example of the three modes (① to ③) of mixed granulated products, it is the only required process for obtaining the desired solid preparation of iodophor to powder the mixed granulated product, and it is not essentially required to mix at least one component selected from the group consisting of dextrin (a), iodides (b), oxidizing agents (c), and acids (d). As a matter of course, it is also acceptable to impose a subsequent process for mixing at least one component selected from the group consisting of dextrin (a), iodides (b), oxidizing agents (c), and acids (d). In another mode, for example, in mode ② [a mixed granulated product containing three components], it is required, for manufacturing the inventive solid preparation of iodophor which contains the four components, to mix at least one component which is absent from the previously mixed granulated product. In the present invention, either is acceptable whether this fourth component alone is added and mixed or is premixed with one or more of the remaining components. The same is applied to the mixed granulated product of mode ① containing two components. That is, for manufacturing the desired solid preparation of iodophor containing the four components, it is required to mix the previously mixed granulated product with the other two components which are absent from the previously mixed granulated product. In another way, these two components to be mixed may be premixed with one or two of the remaining two components. In the present invention, the expression, "if necessary", is used with an intention of including these various modes.

It is particularly recommended that a mixed granulated product contains granulating dextrin (a), together with iodides (b) and/or oxidizing agents (c), under a wet condition produced by spraying water is powdered, mixed with acids (d), and if necessary, further mixed with at least one component selected from the group consisting of dextrin (a), iodides (b), and oxidizing agents (c). It is most recommended that a mixed granulated product comprising wherein granulating dextrin (a), together with iodides (b) and/or oxidizing agents (c), under a wet condition produced by spraying water, powdering the resultant product, and mixing the powder product with acids (d), and if necessary, further with iodides (b) and/or oxidizing agents (c). In this process, it is preferred to spray water containing a binder (pullulan, gum arabic, etc.).

In the following, the liquid bed granulation dryer (Flow Coater) used in the present invention is described by means of FIG. 1 (schematic diagram of the dryer).

First of all, water is introduced into the liquid delivery pump 2, and an appropriate powdered raw materials 1 is placed on the bottom of the Flow Coater. The water delivered by the liquid delivery pump 2 is sprayed on the powdered raw material 1 through the spray nozzle 3. The Flow Coater has been equipped with the bag filter 4 to prevent the powdered raw material 1 escaping from the Flow Coater. The powdered raw material 1 is granulated and dried by supplying intake air (hot air) from the bottom of the Flow Coater to blow the powder up while water is sprayed. Depending on the kind of the powdered raw material, etc., the conditions of mixing, spraying, and drying can be suitably selected within the respective desired ranges.

Within the allowable range of the liquid bed granulation dryer, preferred conditions of spraying and drying are as follows.

### Temperature of intake air:room temperature to 120°C

It is recommended that the lower limit of the temperature of intake air is set at not less than room temperature, more preferably not less than 50°C. Defining the lower limit within this range prevents blocking of the powder (solidification), which may occur because of insufficient drying (spraying excess water), as well as coloration, etc. of the powder because of released iodine, and can also prevent the prolongation of manufacturing time and the decreases in working efficiency. Thus, the cost rising is prevented. Still more preferred is 80°C or more.

On the other hand, the recommended upper limit of the temperature of intake air is 120°C or less, more preferably 100°C or less. At a temperature higher than 120°C, the water sprayed is vaporized within a short time, and thereby powder adsorption is delayed, causing prolongation of manufacturing time, decreases in working efficiency, and rising of the cost. Still more preferred is 95°C or less.

### Wind delivery pressure:100 to 400 mm/Aq

The wind delivery pressure means ones to be applied to make the powder float. It may be adjusted according to the specific gravity or amount of the mixed powder to be loaded, but it is preferred to define the wind delivery pressure within the range from 100 to 400 mm/Aq. When a pressure less than 100 mm/Aq is applied, the powder does not float, and as a result, the water is sprayed only on the upper portion of the loaded volume of the powder, causing blocking (solidification), coloration, etc. of the powder because of released iodine. Still more preferable is 150 mm/Aq or more. At a wind delivery pressure higher than 400 mm/Aq, the powder floats at a level higher than the spray nozzle, so that the water cannot evenly be sprayed on the powder, resulting in granulation failure in some portion of the loaded volume of the powder. This causes some unfavorable phenomena including irregularity in particle size, etc. More preferred is 300 mm/Aq or less, and still more preferred is 250 mm/Aq or less.

### Premixing time:30 minutes or less

The premixing time means the mixing time taken for preparing the granulated mixture. When the premixing time is longer than 30 minutes, the difference in specific gravity of the powder leads to insufficient mixing, lack of uniformity of the resultant mixed granulated product. In addition, the accompanying prolongation of manufacturing time and the decrease in working efficiency make the cost rise. More preferred is 10 minutes or less, and still more preferred is 5 minutes or less. When the premixing time is less than 2 minutes, the resulting insufficient powder mixing causes lack of uniformity of the resultant mixed granulated product, and water spraying on the powder that has not sufficiently been heated causes insufficient drying, which results in blocking of the powder (solidification) or coloration by released iodine. Thus, the premixing time of 2 minutes or more is recommended. More preferred is 3 minutes or more.

### Time for and frequency of water spraying; time for and frequency of interim-drying; and time for finish-drying

These parameters are closely related with each other, and also related to the temperature of the intake air. In considering how excessively water spraying is vaporized (interim-drying), how the water content of the mixed granulated product can be adjusted (finish-drying), whether the cost can be decreased by shortening total manufacturing time, and other things, it is recommended to adjust these parameters within the range described below.

### Time for the post-premixing process: 5 to 40 minutes

When the above process takes less than 5 minutes, insufficient granulation causes lack of uniformity of the resultant particle size, and desired effects cannot be obtained because of the existence of fine particles. More preferred is 10 minutes or more, and still more preferred is 15 minutes or more. When the process takes more than 40 minutes, the cost rises. More preferred is 30 minutes or less, and still more preferred in 20 minutes or less.

### Amount of water to be sprayed:5 to 30 w/w% with respect to the total weight of powdered raw material

The amount of water to be sprayed is closely related with the below time for and frequency of spraying, and additionally may be changed by the temperature of intake air. When the temperature of intake air is high, increased amounts of water can be sprayed, and the time for spraying can be prolonged. In addition, it is also possible to conduct a frequency of water spraying at once. Thus, it is advisable to appropriately adjust the above parameters so as to prevent solidification which may be caused by an excessive content of water spraying in the powder and coloration by released iodine, etc.

It is preferred that the total amount of water spraying is adjust to 5 to 30 w/w% with respect to the total weight of the powdered raw material. When the total amount of water spraying is less than 5 w/w%, insufficient granulation causes lack of uniformity of the resultant particle size, and desired effects cannot be obtained because of the existence of fine particles. More preferred is 10 w/w% or more. However, when the total amount of water spraying is more than 30 w/w%, prolongation of manufacturing time, blocking (solidification) of the powder, coloration by release of iodine, and others occur, decreasing the working efficiency. More preferred is 20 w/w% or less, and still more preferred is 15 w/w% or less.

### Time for and frequency of spraying

The time for and frequency of spraying are to be appropriately adjusted within a appropriate range in consideration of the time for interim-drying (as noted below), etc. It is preferred to spray 1 to 10 times for 1 to 5 minutes each, and more preferred to spray 3 to 5 times for 2 to 3 minutes each.

It is recommended to set the total time for spraying within 5 to 20 minutes. When the total time for water spraying is set at less than 5 minutes, insufficient drying (an excessive content of water spraying) occurs during spraying, causes blocking (solidification) of the powder as well as coloration by released iodine, leading to decreased working efficiency. More preferred is 7 minutes or more. However, when the total time for water spraying is too long, insufficient granulation causes lack of uniformity of the resultant particle size, and desired effects cannot be obtained because of the existence of fine particles. More preferred is 15 minutes or less.

### Time for interim-drying:5 minutes or less

The interim-drying means a preliminary drying process imposed between spraying and drying (finish-drying). Concretely, if spraying is conducted only once in each course of mixing → spraying → drying, then, interim-drying is skipped, and a finish-drying process is conducted once. However, if spraying is conducted 4 times, interim-drying is conducted 3 times, and a finish-drying process is conducted once at last. The interim-drying is distinguished from the finish-drying, which is conducted in order to appropriately adjust the water content before finishing off the final product, by its significance that it prevents the retainment of excessive water, derived from water spraying, in the powder (an excessive water content leads to solidification of the powder and coloration by released iodine, etc.).

The above time for interim-drying can be decided according to the frequency of spraying, etc. as noted above.

However, when the time for interim-drying is too short, insufficient drying (an excessive content of water spraying) leads to blocking (solidification) of the powder or coloration by released iodine, etc., and decreases the working efficiency. Preferred is 30 seconds or more, and more preferred is 1 minute or more. However, when the time for interim-drying is longer than 5 minutes, prolongation of working time makes the cost rise, and desired effects cannot be obtained because of insufficient granulation which causes lack of uniformity of the resultant particle size and the existence of fine particles. In addition, unfavorable phenomena including disintegration of granules occur. More preferred is 3 minutes or less, and still more preferred is 2 minutes or less.

### Time for finish-drying:10 minutes or less

The finish-drying is conducted in order to finally adjust the water content in the mixed granulated product. Too short finish-drying causes insufficient drying which leads to blocking (solidification) of the powder as well as coloration by released iodine. Preferred is 1 minute or more, and more preferred is 2 minutes or more. However, when the time for finish-drying is longer than 10 minutes, prolongation of working time makes the cost rise, and desired effects cannot be obtained because of insufficient granulation which causes lack of uniformity of the resultant particle size and the existence of fine particles. In addition, unfavorable phenomena including disintegration of granules occur. Preferred is 5 minutes or less, and more preferred is 3 minutes or less.

It is preferred that the water content in granulated powder obtained in this manner is 0.5 to 10 w/w%. A water content of less than 0.5 w/w% causes easy disintegration and poor solubility. A water content of more than 10 w/w% increases reactivity with acids, and causes iodide release, leading to easy coloration. More preferred is 5 w/w% or less, and still more preferred is 2 w/w% or less.

The above method is also useful as a method for manufacturing solid preparation of iodophor containing povidone-iodine (a complex of PVP and iodine) and dextrin. Namely, a mixture of them is sprayed with water, granulated in the wet condition, and powdered. Thus, iodine release is inhibited, and stability can markedly be increased. The conditions of mixing, spraying, and drying can appropriately be adjusted according to the mixture ratio of povidone-iodine and dextrin, etc. The preferred conditions are as noted above.

It has been known that povidone-iodine shows a strong antibacterial activity against pathogenic bacteria which infect man, animals, and plants, rapidly kills all species of microorganisms including bacteria, viruses, molds, spore-forming bacteria, protozoa, and yeast, and is also effective against some species of insect, parasite, and nematode. Povidone iodine has been widely used as a bactericide/disinfectant for animals and as a gargle for man, etc. because it has a strong bactericidal activity and a broad antibacterial spectrum as mentioned above, and is also relatively safe for the human body. For example, aqueous solutions of povidone-iodine have been used for various purposes including: disinfectants for barns and devices for livestock farming (sterilization of floors, walls, and ceilings of cow sheds, piggeries, and poultry houses; sterilization of milking devices and incubation devices; post-milking sterilization of nipples; sterilization of hatching egg shells; and others); disinfectants for machines for manufacturing foods and fermentation, etc. (sterilization of facilities and equipment; sterilization of storehouses and refrigerators for components and products; sterilization of containers and packing materials; and others); disinfectants for the wood, etc. nematicide, antisepsis, and anti-microorganism for imported wood; antisepsis and anti-microorganism for leather, paste, textile, paint; and others); environmental disinfectants (sterilization of digester chambers, septic tanks, and water distribution pipes; sterilization of putrefied swamps and ponds); plant disinfectants (antisepsis, anti-microorganism, and insect control for imported plants, tea leaves, and crude drugs; sterilization of storehouses and refrigerators for them; and others); disinfectants for public facilities (sterilization of toilet facilities, wards, and filth facilities in hospitals; pet-related environment; sterilization of the interior of feeding facilities, theaters, public lavatories, public bathes, and vehicles; and others); soil disinfectants; household disinfectants; disinfectants for combs, scissors, etc. used at barber shops/beauty salons; and others.

Thus, povidone-iodine, like molecular iodine, has a strong antibacterial activity and a broad antibacterial spectrum, and does not confer on microorganisms resistance for povidone-iodine. In addition, povidone-iodine has some advantages including less peculiar odor characteristic of iodine, higher safety than that of halogen-type antibacterial agents, and less irritation to humans and animals. On the other hand, the povidone-iodine has the problems because of its poor water solubility, a long time (a few hours to 24 hours) is required to dissolve povidone-iodine in water, that vigorous stirring causes partial ionization of iodine hold in the complex and decreases its bactericidal effect, and a low concentration solutions of povidone-iodine loose its bactericidal effect within a relatively short period of time.

It was found that when fine powder of such povidone-iodine is coated/granulated with fine powder of very highly water-soluble dextrin, its water solubility is highly improved, and that such granulated powder of pvidone iodine/dextrin is completely dissolved in water within a few minutes.

The above granulated powder of povidone-iodine/dextrin is produced by utilizing dextrin for granulating and powdering povidone-iodine, and contains available iodine at a concentration of at least 0.005%. In the concrete, the povidone-iodine contains available iodine at a concentration of 8 to 13 w/w% as PVP-iodine complex, and the weight ratio of povidone-iodine to dextrin in the granulated powder of povidone-iodine/dextrin is preferably 90:10 to 5:95, and especially preferably 80:20 to 10:90.

Water solubility of the povidone-iodine/dextrin powder correlates with the amount of dextrin. Water solubility is higher when the amount of dextrin is larger, and a 10 w/w% or lower content of dextrin makes its solubility poor. At a 5 w/w% or lower content of povidone-iodine, however, its bactericidal effect is deteriorated although its solubility is excellently high. When a fine powder prepared by simply mixing povidone-iodine and dextrin is added in water, povidone-iodine and dextrin separate from each other, so that povidone-iodine comes to float on the water surface and takes a long time for dissolution. However, when granules prepared by granulation of povidone-iodine with dextrin are added in water, they sink in a moment, and completely dissolve within a few minutes.

The present invention is illustrated below in more detail by reference to some examples. However, the following examples do not restrict this invention, and it is all within the contemplation of the present invention to make modifications in the invention unless they deviate from the above- and below tenor.

### PREPARATION EXAMPLE 1

① Potassium iodate was partially reduced with hydrazine, and the powder mixture (740 g) containing the resultant potassium iodide and unchanged potassium iodate in a ratio of 5:1 (by weight) was placed in Flow Coater ("Flow Coater Model FLO-5B", Okawara Seisakusho) together with ② dextrin powder (1260 g, trade name "pinedex #3", Matsutani Kagaku Kogyo), sprayed with water or water containing binders under the below conditions, and granulated and dried, resulting in a powder (weight ratio of ① to ② = 37:63).

### [Conditions for spraying, granulating, and drying]

| | |
|---|---|
| Temperature of intake air | around 90°C |
| Temperature of exhaust gas | 40 to 50°C |
| Wind delivery pressure | 150 to 200 mm/Aq |
| Time for premixing | 5 minutes |
| Time for and frequency of water spraying | 2 minutes and 30 seconds each, 4 times (amount of water spraying 350 mL/4 times) |
| Time for and frequency of interim-drying | 1 minute each, 3 times |
| Time for finish-drying | 5 minutes |

The resultant powder was filtered, and a powder with particle size of around 40 mesh was obtained. This powder was mixed with citric anhydride, sodium hydrogencarbonate, and a surfactant (sodium salt of the condensation product between naphthalensulfonic acid and formalin) in the following ratio:

| | |
|---|---|
| Granulation product containing (KIO₃ + KI) and dextrin | 33 w/w% |
| Citric anhydride | 45 w/w% |
| Sodium hydrogencarbonate | 20 w/w% |
| Surfactant | 2 w/w% |

### PREPARATION EXAMPLE 2

To 10% ethanol aqueous solution (1800 g), 900 g povidone-iodine was added, and dissolved while being heated at 40 to 50°C. On the other hand, 2100 g dextrin fine powder ("Pinedex #6", Matsutani Kagaku Kogyo) was added to water (3150 g), and dissolved under stirring.

Each solutions obtained in this manner were sufficiently mixed respectively, stirred, and homogenized, and then powdered by using a spray drier ("Spray Drier Model L-8", Okawara Seisakusho) (time for spraying 150 minutes, atomizer 25000 r.p.m.) to obtain 2540 g of the resultant powder. A position of 2500 g was taken from this powder, placed in a testing Flow Coater ("Flow Coater Model FLO-5B", Okawara Seisakusho), sprayed with water under the below conditions, and granulated and dried. As a result, the desired povidone-iodine/dextrin granulated powder was obtained.

### [Conditions for spraying and granulating/drying]

| | |
|---|---|
| Temperature of intake air | around 90°C |
| Temperature of exhaust gas | 40 to 50°C |
| Wind delivery pressure | 150 mmHg |
| Time for and frequency of water spraying | 2 minutes and 30 seconds each, 4 times (amount of water spraying 400 mL/4 times) |
| Time for and frequency of interim-drying | 1 minute and 10 seconds each, 3 times |
| Time for finish-drying | 2 minutes |

### EXAMPLE 1 (Stability Test)

In this example, a stability test was performed as described below for the purpose of confirming the excellent stability of the inventive solid preparation of iodophor by comparing some examples of the present invention and reference examples obtained by simple mixing of each component not by using the present invention.

### [Example 1 of the present invention]

First of all, the granulated powder containing potassium iodide (KI), potassium iodate (KIO₃), and dextrin was obtained in the same manner as described in the PREPARATION EXAMPLE 1. This granulated powder was placed in a vinyl plastic bag, and mixed with citric anhydride and sodium hydrogencarbonate placed in the bag.

The resultant Example 1 of the present invention contained each component in the following weight ratio:

| | |
|---|---|
| Granulated powder containing KI, KIO₃, and dextrin | 135.63 g (38.75%) |
| Citric anhydride | 148.71 g (42.49%) |
| Sodium hydrogencarbonate | 65.66 g (18.76%) |

### [Example 2 of the present invention]

The components were processed, granulated, and dried in the same manner as described in PREPARATION EXAMPLE 1, except that a mixed powder containing KI and KIO₃ in a ratio of 6:1 (by weight) was used, and obtained a powder (ratio of ① to ② was 37:63 by weight). To this granulated powder, citric anhydride was added in the following ratio, and mixed.

| | |
|---|---|
| Granulated powder containing KI, KIO₃, and dextrin | 265.5 mg (72.64 %) |
| Citric anhydride | 100.0 mg (27.36 %) |

### [Example 3 of the present invention]

In the Examples 1 and 2 of the present invention, the desired solid preparation of iodophor was obtained by using mixed powders, the ratio of KI to KIO₃ in which was modified by reduction reaction. However, in Example 3 of the present invention, KI and KIO₃ were mixed with each other, and the resultant mixture was used to obtain the desired solid preparation of iodophor. Namely, ① a powder mixture (740.7 g) containing KI and KIO₃ in a ratio of 6:1 (by weight) and ② dextrin powder (1259.3 g, trade name "Pinedex #3", Matsutani Kagaku Kogyo) were placed in the Flow Coater ("Flow Coater Model FLO-5B", Okawara Seisakusho), and sprayed with water in the same manner as described in PREPARATION EXAMPLE 1, granulated and dried, and a powder (weight ratio of ① to ② = 37:63) was obtained. To this granulated powder, citric anhydride was further added in the following ratio, and mixed.

| | |
|---|---|
| Granulated powder containing KI, KIO₃,and dextrin | 265.5 mg (72.64 %) |
| Citric anhydride | 100.0 mg (27.36 %) |

### [Example 4 of the present invention]

The components were processed, granulated, and dried in the same manner as described in PREPARATION EXAMPLE 3, except that ① KI and KIO₃ in a ratio of 8:1(by weight) were mixed with each other, and obtained a powder (ratio of ① to ② was 37:63 by weight). To this granulated powder, citric anhydride was further added in the following ratio, and mixed.

| | |
|---|---|
| Granulated powder containing KI, KIO₃, and dextrin | 341.4 mg (77.34 %) |
| Citric anhydride | 100.0 mg (22.66 %) |

### [Example 5 of the present invention]

The components were processed, granulated, and dried in the same manner as described in PREPARATION EXAMPLE 1, except that ① KI (740.7 g) and ② dextrin powder (1259.3 g, trade name "Pinedex #3", Matsutani Kagaku Kogyo) were used, and obtained a powder (ratio of ① to ② was 37:63 by weight). To this granulated powder, KIO₃, dextrin, and citric anhydride were further added in the following ratio, and mixed.

| | |
|---|---|
| Granulated powder containing KI and dextrin | 227.6 mg (62.27 %) |
| KIO₃ | 14.0 mg ( 3.83 %) |
| Dextrin | 23.9 mg ( 6.54 %) |
| Citric anhydride | 100.0mg (27.36 %) |

### [Example 6 of the present invention]

The components were processed, granulated, and dried in the same manner as described in PREPARATION EXAMPLE 1, except that ① KIO₃ (740.7 g) and ② dextrin powder (1259.3 g, trade name "Pinedex #3", Mateutani Kagaku Kogyo) were used, and obtained a powder (ratio of ① to ② was 37:63 by weight). To this granulated powder, KI, dextrin, and citric anhydride were further added in the following ratio, and mixed.

| | |
|---|---|
| Granulated powder containing KIO₃ and dextrin | 37.9mg(10.37 %) |
| KI | 84.3mg (23.06 %) |
| Dextrin | 143.3mg(39.21 %) |
| Citric anhydride | 100.0mg(27.36 %) |

### [Example 7 of the present invention]

The components were processed, granulated, and dried in the same manner as described in PREPARATION EXAMPLE 1, except that ① citric anhydride (740.7 g) and ② dextrin powder (1259.3 g, trade name "Pinedex #3", Matsutani Kagaku Kogyo) were used, and obtained a powder (ratio of ① to ② was 37:63 by weight). To this granulated powder, KI and KIO₃ were further added in the following ratio, and mixed.

| | |
|---|---|
| Granulated powder containing citric anhydrid | 270.3 mg (73.31 %) |
| KI | 84.3 mg (22.86 %) |
| KIO₃ | 14.1mg (3.82 %) |

### [Example 8 of the present invention]

The components were processed, granulated, and dried in the same manner as described in PREPARATION EXAMPLE 1, except that KI and KIO₃ were mixed in a ratio of 6:1 (by weight), and obtained a powder (ratio of ① to ② was 37:63 by weight). To this granulated powder, KI and citric anhydride were further added in the following ratio, and mixed.

| | |
|---|---|
| Granulated powder containing KI, KIO₃, and dextrin | 265.5 mg (67.45 %) |
| KI | 28.1 mg (7.14 %) |
| Citric anhydride | 100.0 mg (25.41 %) |

### [Reference example 1]

KI, KIO₃, and dextrin were simply mixed not by using Flow Coater. The resultant powder was placed in a widemouthed brown bottle (diameter of the mouth: about 100 mL), and citric anhydride and sodium hydrogencarbonate were further added, and mixed.

The resultant reference example 1 contained the individual components in the following weight ratio:

| | |
|---|---|
| KI | 11.95 g (11.95 %) |
| KIO₃ | 2.39 g (2.39 %) |
| Dextrin | 24.41 g (24.41 %) |
| Citric anhydride | 42.49 g (42.49 %) |
| Sodium hydrogencarbonate | 18.76 g (18.76 %) |

### [Reference example 2]

KI, KIO₃, dextrin, and citric anhydride were mixed, not by using Flow Coater, in the following weight ratio (mixing ratio of KI to KIO₃ = 6:1).

| | |
|---|---|
| KI | 84.3 mg (23.06 %) |
| KIO₃ | 14.1 mg (3.86 %) |
| Dextrin | 167.2 mg (45.75 %) |
| Citric anhydride | 100.0 mg (27.36 %) |

### [Reference example 3]

KI, KIO₃, dextrin, and citric anhydride were mixed, not by using Flow Coater, in the following weight ratio (mixing ratio of KI to KIO₃ = 8:1).

| | |
|---|---|
| KI | 112.4 mg (25.46 %) |
| KIO₃ | 14.1 mg (3.19 %) |
| Dextrin | 215.0 mg (48.70 %) |
| Citric anhydride | 100.0 mg (27.36 %) |

### [Stability test]

The stability test was performed as described below by using the above examples of the present invention and reference examples.

First of all, a 1g portion each was taken from the mixed powders obtained in Example 1 of the present invention and reference example 1, and placed in a transparent-glass sample bottle (a volume of 15 mL). As for the examples 2 to 8 of the present invention and the reference examples 2 and 3, the same amount of each as described above was placed in a transparent-glass sample bottle (a volume of 5 mL). These bottles were closed with plastic caps, wrapped with parafilm, and stored in an incubator maintained at 50°C. On day 1 (example 1 of the present invention and reference example 1) or day 5 (examples 2 to 6 of the present invention and reference examples 2 and 3) of storage, the color was visually observed, and possible iodine release was also examined.

The criteria for the color change and iodine release are as follows:

### Color change

- ○:: unchanged
- △:: white → yellowish white or pale violet
- X:: white → brown to black

### Iodine release

- ○:: iodine not released
- X:: iodine released

The results on day 1 of storage are shown in Table 1, and those on day 5, in Table 2.

**Table 1**

| No. | Color change | Iodine release |
|---|---|---|
| Example 1 of the present invention | ○ | ○ |
| Reference example 1 | △ | X |

**Table 2**

| No. | Color change | Iodine release |
|---|---|---|
| Example 2 of the present invention | △ | ○ |
| Example 3 of the present invention | ○ | ○ |
| Example 4 of the present invention | ○ | ○ |
| Example 5 of the present invention | ○ | ○ |
| Example 6 of the present invention | △ | ○ |
| Example 7 of the present invention | ○ | ○ |
| Example 8 of the present invention | ○ | ○ |
| Reference example 2 | X | X |
| Reference example 3 | X | X |

As shown in Tables 1 and 2, the examples of the present invention were accompanied by neither (or little) color change nor iodine release whereas in the reference examples, marked color changes as well as iodine release were observed.

In order to confirm that iodine had not been inactivated in the examples of the present invention, the samples were dissolved in water after the test was completed. As a result, the color changed into brown, and the peculiar odor of iodine was generated. Thus, it was confirmed that iodine existed availably without inactivation.

### EXAMPLE 2 (water solubility test and antibacterial activity test)

The examples 9 to 11 of the present invention and the reference examples 4 and 5 were prepared according to the following methods.

### [Example 9 of the present invention]

750 g fine powder of povidone-iodine (the content of available iodine is 11.4 % of complex of polyvinylpyrrolidone and iodine, Nippo Kagaku) and 1750 g fine powder of dextrin (Pinedex #3, Matsutani Kagaku Kogyo) were placed in a testing Flow Coater,, and prepared povidone-iodine/dextrin granulated powder containing povidone-iodine and dextrin in a weight ratio of 30:70 (example 9 of the present invention) by the following granulation conditions.

### [Granulation conditions]

| | |
|---|---|
| Temperature of blast inlet | 85°C |
| Temperature of blast outlet | 50°C |
| Wind delivery pressure | 150 mmHg |
| Mixing time | 5 minutes |
| Time for and frequency of water spraying | 2 minutes and 30 seconds each, 7 times (amount of water spraying 500 mL/7 times) |
| Time for and frequency of interim-drying | 1 minute and 10 seconds each, 6 times |
| Time for finish-drying | 3 minutes |

### [Example 10 of the present invention]

The components were processed in the same manner as described in the example 9 of the present invention, except that the amount used was 1000 g for fine powder of povidone-iodine and 1500 g for fine powder of dextrin, and obtained a granulated powder containing povidone-iodine and dextrin in a ratio of 40:60 (example 10 of the present invention).

### [Example 11 of the present invention]

The components were processed in the same manner as described in the example 9 of the present invention, except that the amount used was 1250 g for fine powder of povidone-iodine and 1250 g for fine powder of dextrin, and obtained a granulated powder containing povidone-iodine and dextrin in a weight ratio of 50:50 (example 11 of the present invention).

### [Reference example 4]

The components were processed in the same manner as described in the example 9 of the present invention, except that 2500 g fine powder of povidone-iodine was used without dextrin, and obtained a granulated powder containing 100% povidone-iodine (reference example 4).

### [Reference example 5]

750 g fine powder of povidone-iodine and 1750 g fine powder of dextrin were placed in the testing henschel mixer, mixed for 3 minites, and prepared povidone-iodine/dextrin granulated powder containing povidone-iodine and dextrin in a weight ratio of 30:70 (reference example 5).

Each sample obtained in this manner was tested for water solubility and antibacterial activity as described below.

### [Water solubility]

A 100 g portion of each sample was added into 100 mL water, and stirred gently, and then allowed to stand. The time required for its complete dissolution was measured.

### [Antibacterial activity]

The minimum bactericidal concentration (MBC) of each sample was measured as described below.

Each of 2 kinds of test strain suspension [E. coli (IFM 3039, 5.6 × 10⁸ cells/mL) and MRSA (NTP 2019, 9.0 × 10⁸ cells/mL) was incubated in tryptic soy broth (BBL) at 37°C for 24 hours, and used as the inoculum. Each sample was diluted with water using 2-fold serial dilutions, and used as test solutions.

To each of 10 mL aliquots of these test solutions, 0.1-mL of the inoculum was added, allowed to react for 1 minute, and then iodine remaining in the solution was inactivated by adding an equal volume of 1% sodium thiosulfate aqueous solution. A 0.2-mL aliquot of the inactivated solution was added to 10 mL tryptic soy broth, and incubated at 37°C for 72 hours, and then it was evaluated visually by observing the degree of turbidity of the medium whether the bacterial growth occurred or not.

The results are shown in Table 3.

**Table 3**

| Samples | Water solubility | Antibacterial activity (MBC) | |
|---|---|---|---|
| | | E. coli | MRSA |
| Example 9 of the present invention | Within 2 minutes | 260 ppm | 500 ppm |
| Example 10 of the present invention | Within 3 minutes | 195 ppm | 375 ppm |
| Example 11 of the present invention | Within 5 minutes | 130 ppm | 250 ppm |
| Reference example 4 | 10 hours or more | 65 ppm | 125 ppm |
| Reference example 5 | About 2 hours | 260 ppm | 500 ppm |

The results shown in Table 3 show that the examples 9 to 11 of the present invention were superior in water solubility as well as in antibacterial activity against E. coli and MRSA.

### Industrial Applicability

The present invention was able to efficiently provide a single-type solid preparation of iodophor which is stable because of the composition as described above, releases no iodine even if it is stored at a high temperature, and can rapidly be dissolved before use.

## Claims

1. A solid preparation of iodophor containing:
(a) dextrin,
(b) iodides,
(c) oxidizing agents,
(d) acids.

2. The solid preparation of iodophor according to claim 1, wherein the content of (a) dextrin, (b) iodides, (c) oxidizing agents, and (d) acids in the solid preparations of iodophor is 10 to 95 %, 10 to 50 %, 2 to 10 %, and 5 to 50 %, respectively, by weight.

3. The solid preparation of iodophor according to claim 1 or 2, which contains potassium iodide as the (b) iodide and potassium iodate as the (c) oxidizing agent in a weight ratio of 4 or more:1.

4. A method for manufacturing solid preparations of iodophor containing (a) dextrin, (b) iodides, (c) oxidizing agents, and (d) acids comprising;
granulating the dextrin (a) and at least one component selected from the group consisting of iodides (b), oxidizing agents (c), and acids (d) under a wet condition produced by spraying water,
powdering the resultant mixed granulated product, and
mixing the powder product, if necessary, with at least one component selected from the group consisting of dextrin (a), iodides (b), oxidizing agents (c), and acids (d).

5. The method for manufacturing solid preparations of iodophor according to claim 4, wherein
the granulating step includes granulating the dextrin (a), together with iodides (b) and oxidizing agents (c) under a wet condition produced by spraying water, and
the mixing step includes mixing the powder product with acids (d).

6. The method for manufacturing solid preparations of iodophor according to claim 4, wherein
the granulating step includes granulating the dextrin (a), together with iodides (b) and/or oxidizing agents (c) under a wet condition produced by spraying water, and
the mixing step includes mixing the powder product with acids (d) and, in addition, at least one component selected from the group consisting of dextrin (a), iodides (b), and oxidizing agents (c), if necessary.

7. The method for manufacturing solid preparations of iodophor according to one of the claims 4 to 6, wherein
the content of (a) dextrin, (b) iodides, (c) oxidizing agents, and (d) acids in the solid preparations of iodophor is 10 to 95 %, 10 to 50 %, 2 to 10 %, and 5 to 50 %, respectively, by weight.

8. The method for manufacturing solid preparations of iodophor according to claim 7, wherein
the granulating step includes granulating at a temperature of intake air ranging from a room temperature to 120°C.

9. The method for manufacturing the solid preparations of iodophor according to claim 7 or 8, which contain potassium iodide as the (b) iodide and potassium iodate as the (c) oxidizing agent in a weight ratio of 4 or more:1.

10. Solid preparations of iodophor that is a granulated powder containing povidone-iodide and dextrin.

11. The solid preparation of iodophor according to claim 10, wherein the content of available iodine in the solid preparations of iodophor is 0.005 w/w% or more.

12. The solid preparation of iodophor according to claim 10 or 11, which contains the povidone-iodine and dextrin in a weight ratio of 90:10 to 5:95.

13. The method for manufacturing solid preparations of iodophor according to one of the claims 10 to 12, wherein the povidone-iodine and dextrin are granulated under a wet condition produced by spraying water.

14. The method for manufacturing solid preparations of iodophor according to claim 13, wherein the granulated powder is obtained at a temperature of intake air ranging from a room temperature to 120°C.
